# EUROPEAN PATENT APPLICATION

(11) **EP 3 685 868 A1**
(43) Date of publication of application: **29.07.2020**
(21) Application number: 20162484.8
(22) Date of filing: 09.01.2018
(51) Int. Cl.: A61M 5/14, A61M 5/142, A61M 5/145, A61M 5/20, A61M 5/32, A61M 5/155, A61M 5/158, A61M 5/19, A61M 5/24, A61K 9/00, A61M 37/00, A61M 5/172

(54) **SYSTEMS AND METHODS FOR WEARABLE EMERGENCY DRUG INJECTION DEVICES**

(30) Priority: 09.01.2017 US 201762444237 P; 12.05.2017 US 201762505457 P
(62) Divisional of application: 18150703.9
(71) Applicant: Verily Life Sciences LLC, Mountain View, CA 94043 (US)
(72) Inventor: KRASNOW, Benjamin, Mountain View,, CA California 94043 (US); WHITEHURST, Todd, Mountain View,, CA California 94043 (US); GLASSMAN, Ethan, Mountain View,, CA California 94043 (US); PARLIKAR, Tushar, Mountain View,, CA California 94043 (US); CHEN, Michael, Mountain View,, CA California 94043 (US); LUI, Clarissa, Mountain View,, CA California 94043 (US)
(74) Representative: Mewburn Ellis LLP

(57) **Abstract**

One example device includes a housing defining at least one chamber and an injector disposed within the housing and comprising a hollow needle corresponding to a piston, the piston disposed and translatable within the chamber towards a first end of the chamber, the piston defining a void intersecting a hollow portion of the hollow needle, a needle guide configured to bend the hollow needle in response to movement of the hollow needle through the needle guide; and a propellant disposed within the chamber and positioned to force the piston towards the first end of the chamber in response to activation of the propellant.

## Description

### FIELD

The present application generally relates to drug injection devices, and more specifically relates to systems and methods for wearable emergency drug injection devices

### BACKGROUND

People with certain medical conditions may require doses of medication in response to certain physiological conditions. For example, a diabetic may monitor her blood sugar and, if it gets too high, inject insulin to help lower the blood sugar levels. Conversely, she may eat some food if her blood sugar gets too low. Another example is a person with an allergy to peanuts or insect stings that experiences anaphylaxis as a result of contact with the allergen. To respond to the anaphylaxis, the person may inject herself with epinephrine, such as with an off-the-shelf epinephrine injector, *e.g.,* an EpiPen®.

### SUMMARY

Various examples are described for systems and methods for wearable emergency drug injection devices. In accordance with the present invention, there is provided a wearable device for injecting a substance according to claim 1.

These illustrative examples are mentioned not to limit or define the scope of this disclosure, but rather to provide examples to aid understanding thereof. Illustrative examples are discussed in the Detailed Description, which provides further description. Advantages offered by various examples may be further understood by examining this specification.

### BRIEF DESCRIPTION OF THE DRAWINGS

The accompanying drawings, which are incorporated into and constitute a part of this specification, illustrate one or more certain examples and, together with the description of the example, serve to explain the principles and implementations of the certain examples.
Figures 1A-1D show an example wearable emergency drug injection device according to this disclosure;
Figure 2 shows an example system for wearable emergency drug injection devices according to this disclosure;
Figures 3A-3G show an example wearable emergency drug injection device according to this disclosure;
Figures 4A-4B and show example wearable emergency drug injection device according to this disclosure;
Figures 5A-5B show an example wearable emergency drug injection device according to this disclosure;
Figures 6A-6D show an example wearable emergency drug injection device according to this disclosure;
Figures 7A-7B show an example wearable emergency drug injection device according to this disclosure;
Figure 8 shows an example wearable emergency drug injection device according to this disclosure; and
Figure 9 shows an example method for using a wearable emergency drug injection device according to this disclosure.

### DETAILED DESCRIPTION

Examples are described herein in the context of systems and methods for wearable emergency drug injection devices. Those of ordinary skill in the art will realize that the following description is illustrative only and is not intended to be in any way limiting. Reference will now be made in detail to implementations of examples as illustrated in the accompanying drawings. The same reference indicators will be used throughout the drawings and the following description to refer to the same or like items.

In the interest of clarity, not all of the routine features of the examples described herein are shown and described. It will, of course, be appreciated that in the development of any such actual implementation, numerous implementation-specific decisions must be made in order to achieve the developer's specific goals, such as compliance with application- and business-related constraints, and that these specific goals will vary from one implementation to another and from one developer to another.

A person with a medical condition, such as diabetes or a severe allergy to a substance, may use a wearable emergency drug injection device according to this disclosure. In this example, the person (also the "wearer") obtains the device, which is approximately an inch wide, one and a half inches long, and half an inch tall. The example device has two halves that clip together to form the completed device.

One half, the disposable half, has components to store and deliver a dose of an injectable substance, *e.g*., 1 milligram ("mg") of glucagon powder and 1 millilitre ("ml") of an activation solution that when mixed with the glucagon, activates the glucagon to enable it to be metabolized by the wearer. Specifically, the disposable half has two chambers. Each chamber has a piston that initially is at one of the chamber. Within each chamber is one of the two substances - the glucagon powder or the activation solution. In addition, one of the two chambers has a component with a hollow needle attached to it, which enables injection of the substances into the wearer. Behind each of the pistons is a small charge that, when activated, generates pressure behind the piston to force the piston to the opposite end of the chamber, thereby expelling the contents of the respective chamber.

The second half of the device, the reusable half in this example, includes circuitry to receive a command to inject the injectable substance and to activate the two small charges in response to the command. For example, the wearer could press a button on the reusable half to trigger the circuitry to activate the charges. Alternatively, the circuitry could receive the command wirelessly from another device, such as the wearer's smartphone, CGM, insulin pump, etc.

In this example, the circuitry is configured to activate the two charges in sequence. The first charge forces the piston towards the end of the chamber that has the needle, which forces the needle to extend out of the device and bend towards and into the wearer's skin. As mentioned above, the needle is hollow and is exposed to a small void or cavity within the piston, in which the glucagon has been deposited. Thus, after the first charge is activated, the needle is inserted into the patient, but the glucagon powder remains within the void in the piston.

After the first charge has activated, the second charge is activated, which forces the piston in the second chamber towards to opposite end of the chamber. The piston's movement forces the activation solution out of the second chamber, through the void in the first piston, where it mixes with the glucagon powder, and then into the hollow needle and ultimately into the patient. Thus, a dose of glucagon is delivered to the patient in response to, for example, a continuous glucose monitor ("CGM") detecting a low glucose level and transmitting a signal to the device.

This illustrative example is given to introduce the reader to the general subject matter discussed herein and the disclosure is not limited to this example. The following sections describe various additional non-limiting examples and examples of systems and methods for wearable emergency drug injection devices.

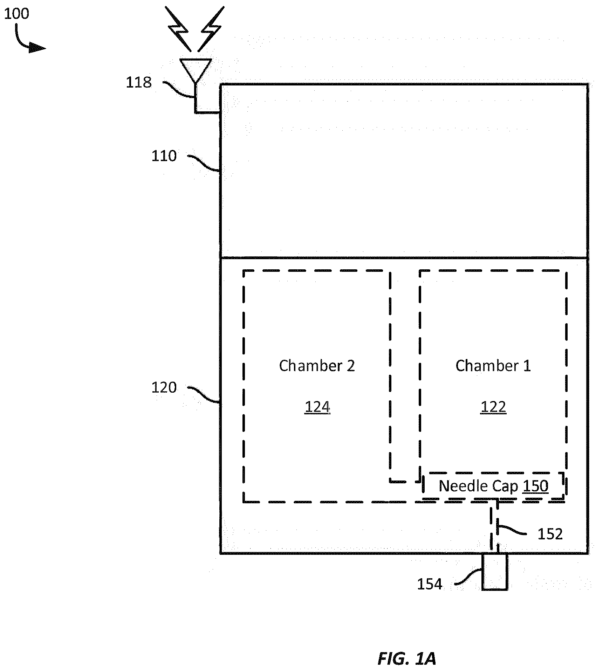

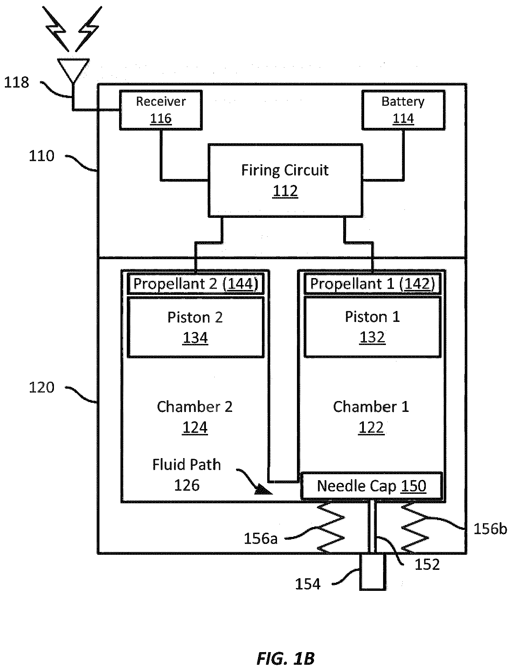

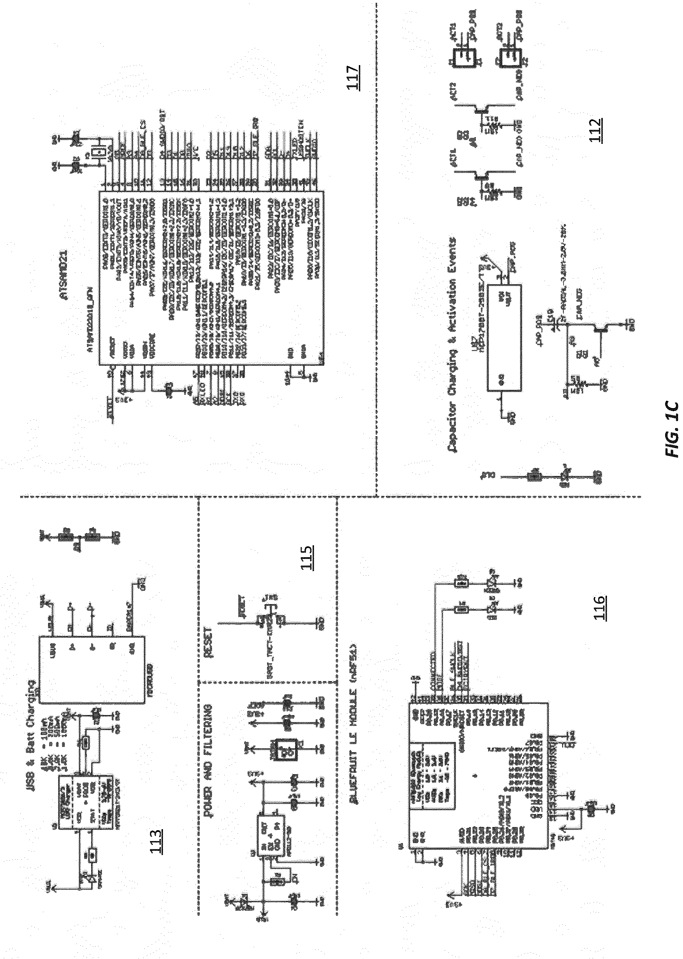

Referring now to Figure 1A, Figure 1A shows an example wearable emergency drug injection device 100. As can be seen in Figure 1A, the example device 100 has two portions 110, 120 that are connected, but are separable from each other. The first portion 110 has electronic components within it, which are described in more detail with respect to Figures 1B and 1C, and an antenna 118 to receive wireless signals. The first portion 110 in this example is separable from the second portion 120 to allow for re-use of the electronics, while the second portion can be discarded after it has been used.

The second portion 120 has two chambers that can be used to store injectable material(s), as well as a hollow needle 152 and a needle cap 150 that can be used to drive the needle 152 through the needle guide 154 and into a person's skin. In this example, because the needle 152 is hollow, injectable material(s) can be forced out of one or both chambers, through the needle, and into the wearer.

The example device shown in Figure 1A is designed to be worn flush against a wearer's body, such as on an upper arm or torso. The needle 152, as shown in Figure 1A, is oriented to extend parallel to the wearer's skin; however, the needle guide 154 defines a curved path that forces the needle 152 to bend towards the wearer's skin at an angle departing from its initial orientation by approximately 30 degrees in this example. Thus, the needle 150, in this example, is formed of a flexible materials, such as a nickel-titanium alloy (*e.g*., Nitinol), to allow the needle 152 to bend at angles of up to 30 degrees (or more) without breaking or obstructing the fluid path through the interior of the needle 152. In addition, the needle 152 in this example is a 22-gauge needle. Such a needle size may provide a diameter suitable for injecting fluid into the wearer while having a diameter that causes a tolerable amount of discomfort to the wearer; however, other suitable needle diameters may be employed.

With respect to description of length, width, and height, the height of the device 100 shown in Figure 1A refers to how far the device extends above the wearer's skin when worn as described above. The length and width, by contrast, refer to the dimensions of the perimeter of the device 100 shown in Figure 1A.

Referring now to Figure 1B, Figure 1B shows a more detailed view of the interior of the first and second portions 110, 120 of the device 100. As discussed above, the second portion 120 defines two chambers 122, 124. Within each chamber 122, 124 is a piston 132, 134 which are initially positioned at one end of the respective chamber opposite an opening. Thus, when the pistons 132, 134 move, the contents of the corresponding chamber 122, 124 are expelled through their respective opening.

The pistons 132, 134 are sized to have approximately the same cross-sectional area as the corresponding chamber 122, 124 to prevent the contents of the chamber 122, 124 from sliding around the piston or, as will be described above, gas pressure generated behind the piston from being dissipated by escaping around the piston 132, 134. In addition, in some examples, one or more of the pistons 132, 134 may have one or more ring seals attached around the perimeter of the piston 132, 134 to prevent such leakage of material or gasses past the piston 132, 134.

A propellant 142, 144 is disposed behind each piston 132, 134. When one of the propellants 142, 144 is activated, it generates pressure within the portion of the chamber behind the piston 132, 134, thereby forcing the piston 132, 134 towards the opposite end of the chamber.

In this example, each propellant 142, 144 comprises a nitrocellulose material, and propellant 1 (142) has a faster-burning nitrocellulose material than propellant 2 (144). For example, propellant 1 (142) in this example is a nitrocellulose in a cotton-based format, while propellant 2 (144) in this example is a nitrocellulose in a paper-based format. Selection of an appropriate propellant may be made based on the contents of the chamber.

For example, chamber 1 may have no injectable material in it, or may have an amount of an injectable powder or liquid, and thus may provide a mechanism for forcing the needle cap 150 and needle 152 downwards, thereby injecting the needle into the wearer's skin. In such an example, a faster-burning propellant may be used as concerns about over-pressurizing the chamber 122 may be reduced. In contrast, in this example, chamber 2 has an injectable fluid. Thus, a slower-burning or slower-acting propellant may be desired to allow time for the fluid to be expelled from the chamber 122 without over-pressurizing the chamber walls. In addition, selection of propellants may be made based on a desired firing sequence, a time to deliver a full dose of material to the wearer, or a time between insertion and retraction of the needle 152.

In some examples, a quantity of propellant for a chamber may be made up of multiple discrete propellant elements, each of which may be individually activatable. Thus, to activate the propellant, each individual propellant element may be activated separately or in combination. In examples where the individual propellant element is activated separately, a firing sequence may be employed to activate the propellant elements to create a desired pressure curve over time. For example, the propellant elements may be activated at regular intervals, *e.g*., one ever half-second, or several may be activated initially to create a high pressure, *e.g.,* to drive a needle into the wearer's skin, followed by successive activation of the remaining propellant elements to slowly and steadily inject a substance into the wearer.

To enable the injectable material to move from the chamber(s) into the wearer, as discussed above, the needle 152 is hollow. In addition, a fluid path 126 is defined between the two chambers to allow injectable material to move from chamber 2 (124) through the fluid path 126 over the needle cap 150 and into the needle 150. And while it is referred to as a "fluid" path 126, it can allow solid (*e.g*., powders) or gaseous materials to flow as well. In addition, piston 1 (132) also defines a void that, after piston 1 (132) has been driven to the opposite end of the chamber 122, the void is exposed to the fluid path as well as the hollow portion of the needle. Thus, the combination of the fluid path 126, the void within piston 1 (132), and the hollow needle 152 provide a path for an injectable material to be expelled from the chamber(s) 122, 124 and into the wearer.

In addition, in this example, a pair of springs 156a-b is coupled to the needle cap to enable retraction of the needle 152. Thus, after the injectable substance has been expelled out of the chamber(s) and in to the wearer, the device 100 may retract the needle 152, via the springs 156a-b in this example. For example, the pressure generated by propellant 1 (142) may initially overcome the spring force, but as the pressure dissipates, *e.g*., via an exhaust port, the springs 156a-b may ultimately overcome the pressure and retract the needle 152. In other examples, other needle retraction mechanisms may be employed, such as another propellant charge located beneath the needle cap.

While the second portion 120 includes the injectable material(s) and the mechanisms for inserting the needle 152 into the wearer and for storing and expelling the injectable material(s), the first portion 110 includes components to receive a command (or commands) to activate the propellant and inject the injectable material(s). In this example, the first portion 110 includes a firing circuit 112, a battery 114 or other electrical power source or connection, a wireless receiver 116, and an antenna 118. To activate the propellants 142, 144 and inject the injectable material into the wearer, in this example, a command is received via the antenna 118 and the receiver 116 from a remote device, such as the wearer's smartphone or a biosensor (*e.g*., a CGM), and is provided to the firing circuit 112. In response to receiving the command, the firing circuit 112 activates the propellants 142, 144 using power supplied by the battery 114.

In this example, the propellants 142, 144 are activated by an electrical discharge. To supply the electrical discharge, the firing circuit 112, prior to receiving the command in this example, charges two capacitors using the battery 114. Upon receiving the command from the receiver 116, the firing circuit 112 couples the capacitors, in sequence, to electrical leads in contact with the respective propellant 142, 144, thereby allowing them to discharge and activate the corresponding propellant 142, 144.

An example of the firing circuit 112 is shown in Figure 1C. As described above, the firing circuit 112 in this example includes capacitors selectably connected to a corresponding propellant charge. By closing a corresponding switch, *e.g*., a transistor, the capacitor's charge is delivered to the propellant 142, 144, activating it. For example, the charge may be applied to a resistor in contact with the propellant. The charge may cause the resistor to generate heat, which ignites the propellant.

In addition to the firing circuit 112, other electronic components may be provided within the first portion 110 as well, such as battery charging circuitry 113, which may a wired connection or a wireless power antenna and rectifier, power and filtering circuitry 115, and a microcontroller 117, *e.g.,* an ASIC defined on a field-programmable gate array ("FPGA"). Still further electronic components may be included within the first portion 110 to enable various features according to this disclosure.

While this example employs a wireless command to activate the firing circuit 112, in some examples, the device 100 may instead have a wired connection to another device, *e.g.,* a biosensor, or may have a button or other wearer manipulatable device ("manipulandum") to activate the firing circuit 112.

Further, while the example shown in Figure 1A-1B has two portions 110, 120 that may be decoupled from each other, in some examples, the device 100 may be formed from a single portion that includes the components described above, or other components according to this disclosure. Thus, rather than providing a second portion 120 that is disposable and first portion 110 that is reusable, the entire device may be discarded.

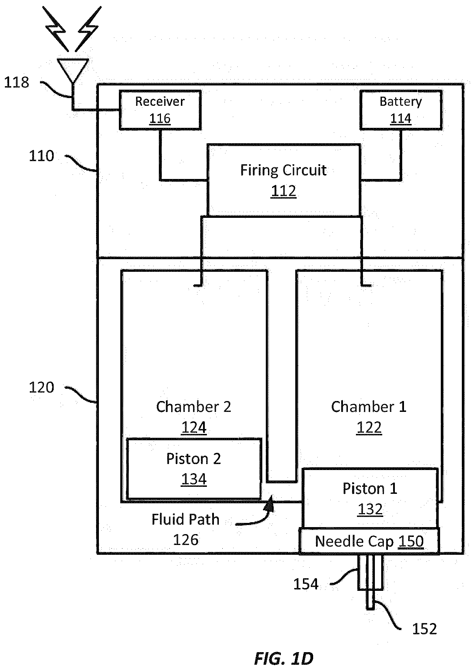

Referring now to Figure 1D, Figure 1D shows the device 100 after the propellants 142, 144 have been activated and the pistons 132, 134 driven to the opposite end of the chambers 122, 124. Piston 1 (132) has driven needle cap 150 and needle 152 away from the bottom of the first chamber 122. The needle 152 has travelled through the needle guide 154, where it was bent towards the wearer's skin. The second piston 134 has expelled the contents of chamber 2 through the fluid path 126, the void in piston 1 (132), and the needle 152 into the wearer.

While in this example, piston 2 (134) has been driven the full length of chamber 2, in some examples, the piston 134 may only be driven part of the length of the chamber. For example, propellant sufficient to only drive the piston partway through a chamber, or one or more physical obstructions may be disposed within the chamber 124, or formed in the walls of the chamber 124 to prevent the piston from travelling the full length of the chamber. Such a feature may be desirable to allow for a greater quantity of injectable material to be disposed within the chamber 124 than is to be dispensed in a single dose. For example the chamber 124 may store 1 ml of epinephrine, but one or more obstructions may permit the piston to expel only, for example, 0.3 ml of epinephrine. Such features may enable a more uniform manufacturing process or help ensure sufficient injectable material is injected, even in the event of a partial failure of the device, *e.g.,* the propellant 144 only partially activates.

While the example device 100 shown in Figures 1A-1D has two chambers, any suitable number of chambers may be employed. For example, an example wearable emergency drug injection device may only have a single chamber, such as chamber 1 shown in Figure 1B. Or a second portion 120 may have more than two chambers, each configured with a piston and propellant. Further, the device may include multiple needles to enable delivery of multiple doses, or doses of different types of injectable materials based on a received command.

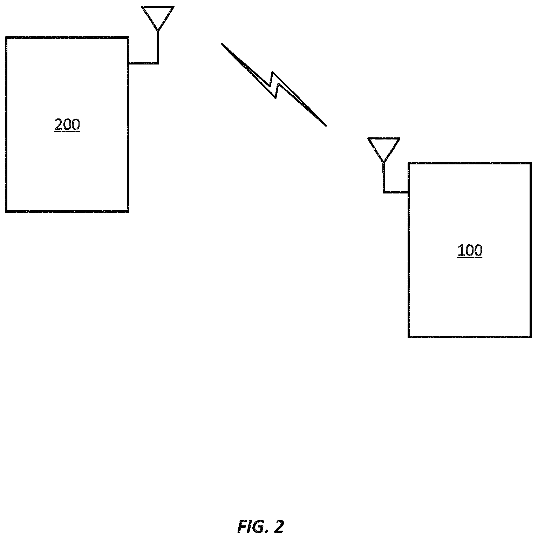

Referring now to Figure 2, Figure 2 shows an example system for a wearable emergency drug injection device. In this example, the device 100 shown in Figures 1A-1D receives a wireless command from remote device 200. The remote device 200, as described above, may be any suitable device with a wireless transmitter, such as a smartphone, smartwatch, blood pressure sensor, CGM, etc. Such remote devices may be handheld or wearable devices or larger devices, such one or more sensing systems as may be found in a hospital or other medical office. Suitable wireless communication mechanisms include Bluetooth®, Bluetooth® low-energy ("BLE"), WiFi, near-field communications ("NFC"), etc.

In one example, the remote device 200 is a CGM 200 that senses and stores glucose levels over time for the wearer. The glucose levels may be accessed wirelessly by various devices, such as the wearer's smartphone, an insulin pump, or example wearable emergency drug injection devices according to this disclosure. In this example, the CGM 200 is configured with a glucose level threshold, below which the wearer is experiencing a hypoglycemic event. The CGM 200 may periodically measure the wearer's glucose levels and compare them to the glucose level threshold. If a measured glucose level (or several consecutive measured glucose levels) falls below the glucose level threshold, the CGM 200 may determine a hypoglycemic event. In this example, the CGM 200 may issue an alert to the wearer, such as by transmitting a signal to the wearer's insulin pump to trigger an audible alarm. The CGM 200 may also transmit a signal to the device 100 to cause it to deliver a dose of glucagon to the wearer.

In this example, the CGM 200 first transmits a signal to the wearer's insulin pump, if the wearer has one, and continues to monitor the wearer's glucose levels to detect whether they rise above the glucose level threshold. If the glucose levels rise above the glucose level threshold, it may indicate that the wearer has eaten some food and the hypoglycemic event has passed. However, if after a predetermined period of time, *e.g.,* 5 minutes, the hypoglycemic event continues or worsens, the CGM 200 may then determine intervention is needed and transmit the signal to the device 100 to cause a dose of glucagon to be injected into the wearer. Such an example may be desirable as it may allow the wearer to raise their glucose levels, even if they are unresponsive, *e.g.,* due to being asleep or unconscious. And while this example relates to a hypoglycemic event and a CGM, other biosensors may be employed as well or instead.

For example, blood pressure, ECG, blood oxygen, etc. biosensors may be employed in some examples to detect medical events, such as anaphylaxis, etc., which may then trigger the biosensor, or another device such as a smartphone, to transmit a signal to the device 100 to cause an injectable material, *e.g*., epinephrine, naloxone, etc., to be injected into the wearer. Thus, different medical events may be addressed or mitigated automatically via the combination of the remote device 200 and the example wearable emergency drug injection device 100, which may address an emergency condition or may allow time for a full medical response to occur, if needed.

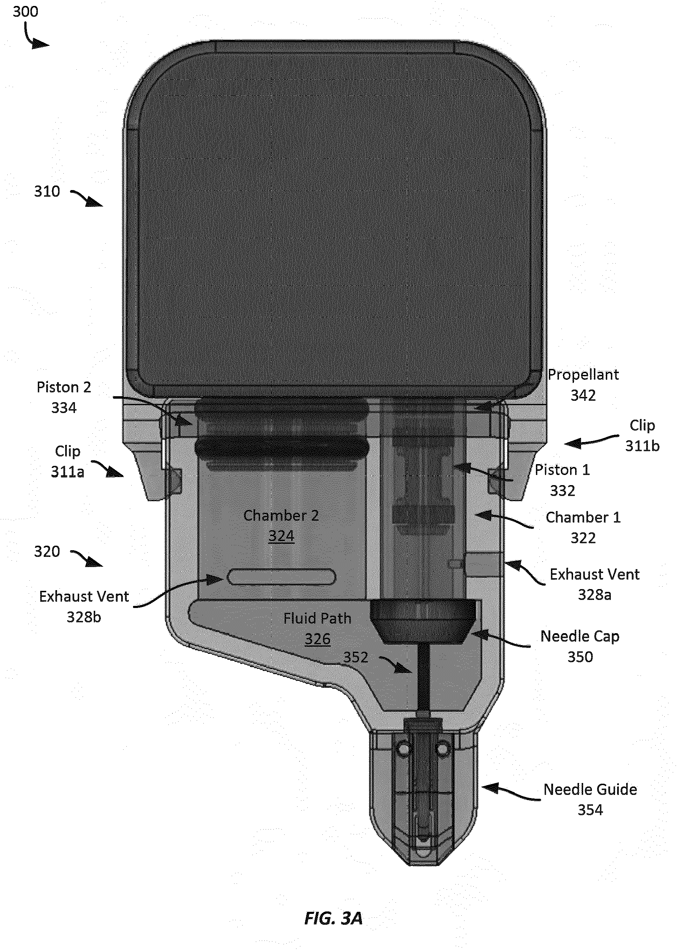

Referring now to Figures 3A-3G, these figures show different views of an example wearable emergency drug injection device 300. Referring to Figure 3A, the wearable emergency drug injection device 300 (or device 300) includes two portions, an electronics portion 310 and an injection portion 320. As can be seen, the electronics portion 310 is releasably coupled to the injection portion 320 by two clips 311a-b. The clips 311a-b engage with the injection portion 320 to releasably secure the two portions 310, 320 together.

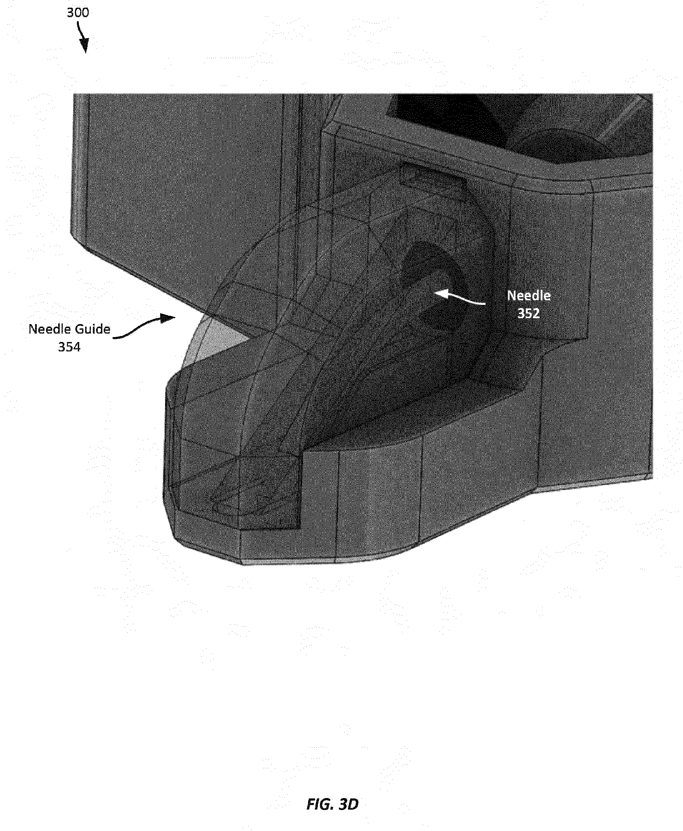

With respect to the injection portion 320, it defines two chambers 322, 324, within each of which is a corresponding piston 332, 334. In addition, the injection portion 320 defines a fluid path 326 that can provide a fluid coupling between chamber 2 (324) and a void (shown in Figure 3D (329)) defined in piston 1 (332) after piston 1 (332) has been driven to the opposite end of the chamber 322. Figure 3A shows the device 300 prior to injecting injectable material, thus, the pistons 332, 334 are positioned at one end of the chamber. Each chamber also includes propellant 342 positioned behind the corresponding piston 332, 334, though note that propellant 344 behind piston 2 (334) is not visible in this figure.

As discussed above, one or both of the chambers may have an injectable substance contained within it. For example, suitable injectable substances may include epinephrine, naloxone, glucagon or a glucagon activation solution, or other drugs or chemicals.

Each of the chambers 322, 324 has an exhaust vent 328a-b to allow gasses generated by the propellant 342, 344 to escape from the respective chamber 322, 324. And while in this example, the exhaust vents 328a-b vent gasses directly into the wearer's environment, in some examples, one or more exhaust vents 328a-b may vent exhaust gasses into a needle retraction mechanism.

The injection portion also includes a needle cap 350 and a hollow needle 352 that are arranged within the fluid path 326, and a needle guide 354. The needle cap 350 provides two functions in this example device. First, the needle cap 350 provides a coupling surface and way to transfer force from piston 1 (332) to the needle 350 to drive the needle 350 through a path in the needle guide 354 and into the wearer's skin. The needle cap 350 also provides a fluid barrier between the fluid path 326 and chamber 1 (322) prior to activation of the propellant 342 behind piston 1 (332).

The needle 352 in this example is hollow to allow injectable material to flow through the needle 352 and into the wearer. In addition, the needle 352 is constructed from a flexible material, such as a suitable plastic or metallic material, such as Nitinol. The needle 352 in this example is sufficiently flexible that it can bend at an angle of between 30 to 45 degrees without permanently deforming and while maintaining a fluid path through the needle.

The needle guide 354 is formed in or coupled to the injection portion 320 to provide a path through which the needle 352 is forced to bend at an angle towards the wearer's skin. Thus, as the needle 352 is driven by the piston 332, it moves into and through the path formed in the needle guide 354 and bends towards the wearer's skin.

As discussed above with respect to Figures 1A-1D, while example devices according to this disclosure may include needle retraction mechanisms, this example device 100 does not include such a feature. However, as discussed above, any suitable needle retraction mechanism, including springs or an exhaust gas retraction arrangement may be employed according to various examples.

Referring now to the electronics portion 310, the electronics portion 310 houses one or more electronic circuits to receive an activation signal, such as wirelessly as described above with respect to Figures 1A-1D and 2, or from a wearer interaction with a manipulandum. In this example, the electronics portion 310 includes the example electronic circuits discussed above with respect to Figure 1C and 1D, which may be used to activate the device's propellant 342,344.

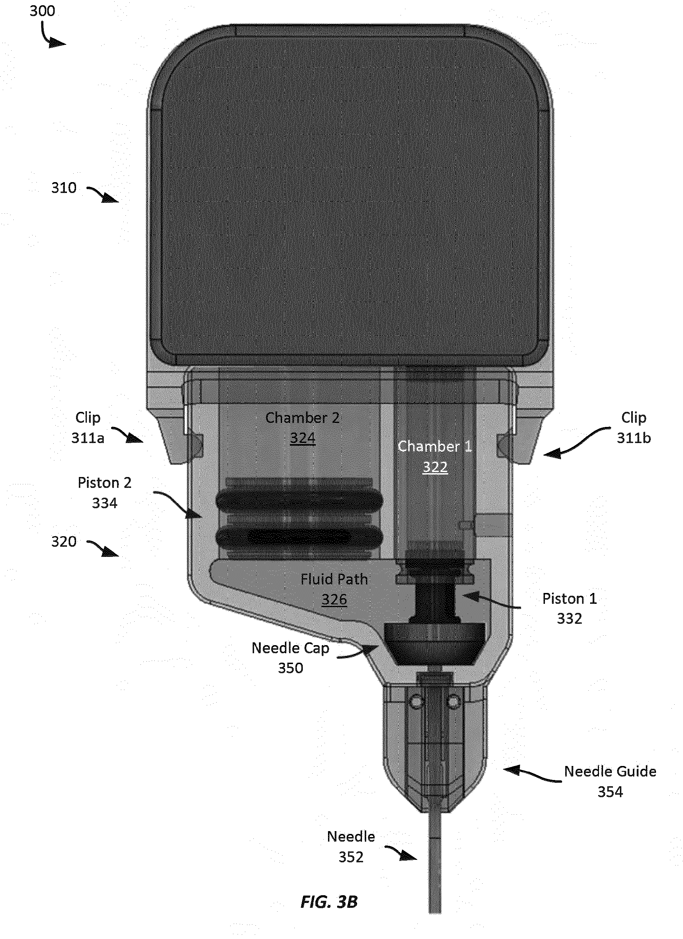

Referring now to Figure 3B, Figure 3B shows a view of the device 300 of Figure 3A after the propellant 342, 344 has been activated and the pistons 332, 334 have been driven to the opposite end of their respective chambers 322, 324. In this example, propellant 342 was activated first, which forced piston 1 (332) to the opposite end of chamber 1 (322) and against the needle cap 350, thereby forcing the needle cap 350 and needle 352 away from the chamber 322 and the needle 352 through the needle guide 354 and into the wearer's skin.

After piston 1 (332) completed its movement, the second propellant 344 was activated, driving piston 2 (334) to the opposite end of chamber 2 (324), thereby forcing any injectable substance in chamber 2 (324) through the fluid path 326, the void 329 in piston 1 (332), and the needle 352 into the wearer. In this example, as discussed above, piston 1 (332) also defines a void through which the injectable substance from chamber 2 flows, thereby enabling the injectable substance to mix with another injectable substance prior to flowing through the needle 352 and into the wearer. Such a configuration may enable the use of substances that may be activated by mixing them prior to injection into the wearer.

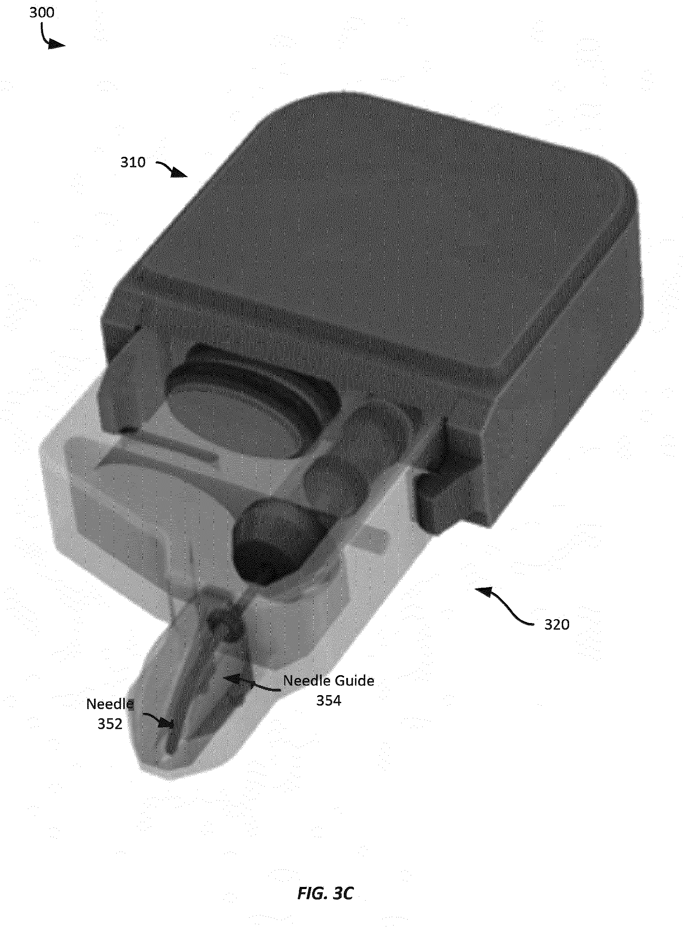

Referring now to Figures 3C and 3D, Figures 3C and 3D show perspective views of the device 300 of Figures 3A-3B. These perspectives show the shape of the needle guide and the bend formed in the needle 352 as it travels through the needle guide 354.

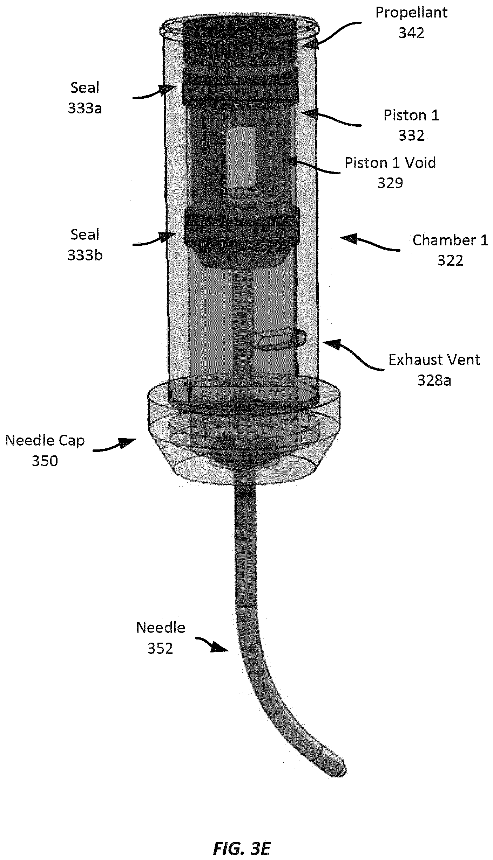

Referring now to Figure 3E, Figure 3E shows the components corresponding to chamber 1 (322) discussed above with respect to Figure 3A. As can be seen, the piston defines a void 329 with a small hole to provide a fluid path through the void 329 and into the needle 352. In this example, the needle 352 extends into the chamber 322 and couples to the piston 332 such that the piston 332 directly drives the needle 352. However, in some examples, the needle 352 may not extend into the chamber 322. In some such examples, the hole formed in the piston 332 may be aligned with the needle 352 to create a fluid path through the void 329 to the needle 352 after piston 1 (332) has been driven into contact with the needle cap 150. Further, this figure illustrates piston seals 333a-b that create a seal between the piston 332 and the chamber wall to help prevent exhaust gasses from the propellant 342 from passing around the piston 332 and into the chamber 322. Similar seals may be provided on piston 2 (334) as well.

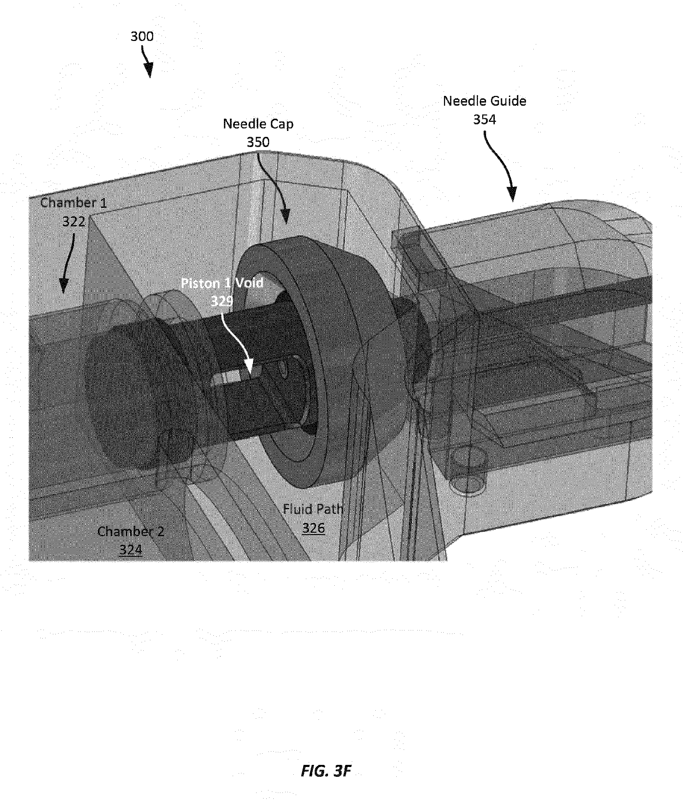

Figure 3F shows a perspective view of the device 300 of Figures 3A-3B after piston 1 (332) has been driven to the opposite end of the chamber 322 following activation of the propellant 342. As can be seen, the void 329 in piston 1 (332) is exposed to the fluid path 326 and allows injectable material to be expelled from chamber 2 (324) through the fluid path 326 and void 329 into the needle 352, and subsequently into the wearer.

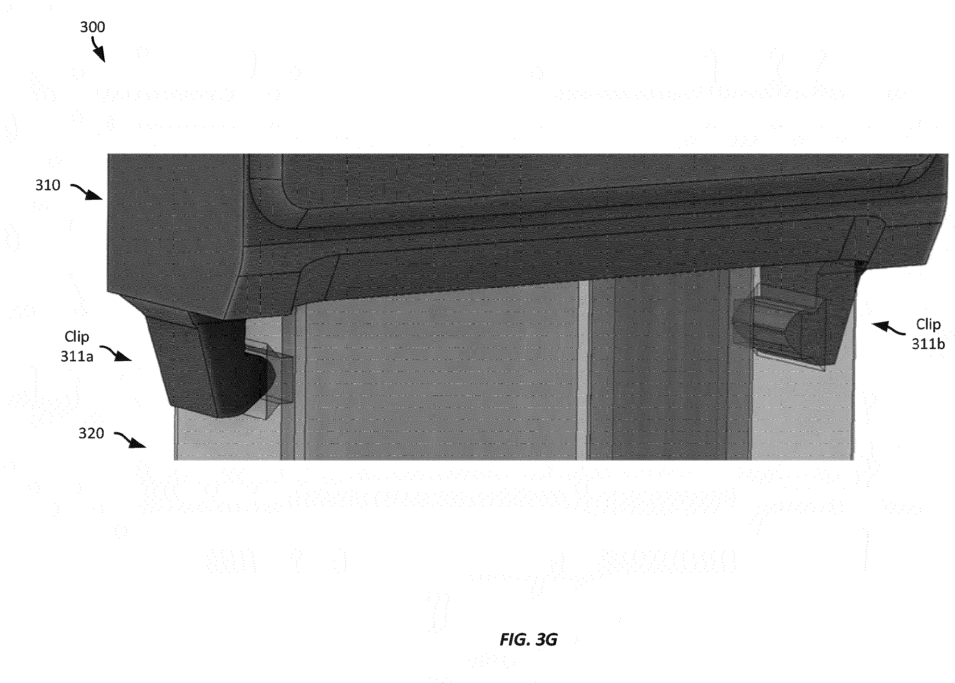

Figure 3G shows a close-up perspective view of the clips 311a-b used to secure the electronics portion 310 to the injection portion 320.

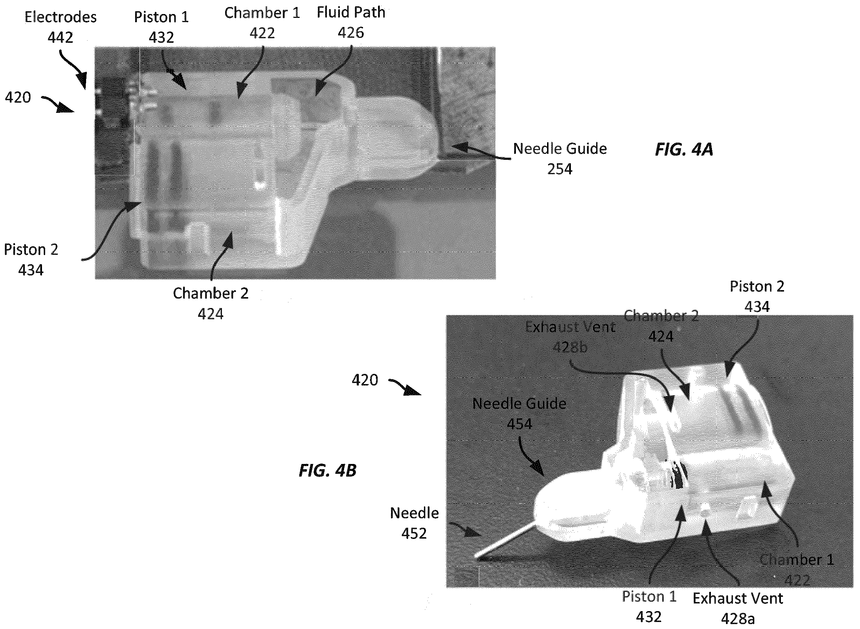

Referring now to Figures 4A-4B, these figures show a further example wearable emergency drug injection device according to this disclosure. In this example, only the injection portion 420 of the device is shown. The injection portion 420 shown in Figure 4A illustrates the device before the propellant has been activated, while Figure 4B shows the device after the propellant behind piston 1 (432) has been activated.

The injection portion 420 defines two chambers 422, 424, each of which has a piston 432, 434 generally as described above, and each chamber has an exhaust vent 428a-b defined in the chamber wall to allow combustion gasses to escape the respective chamber 422, 424. In addition, the injection portion 420 defines a fluid path 426 that provides a fluid coupling between chamber 2 (424) and the needle 452 after the first piston 432 has been driven to the opposite end of the chamber 422. The injection portion 420 also includes a needle guide 454, which as discussed above, causes the needle 452 to bend as it traverses the needle guide 454.

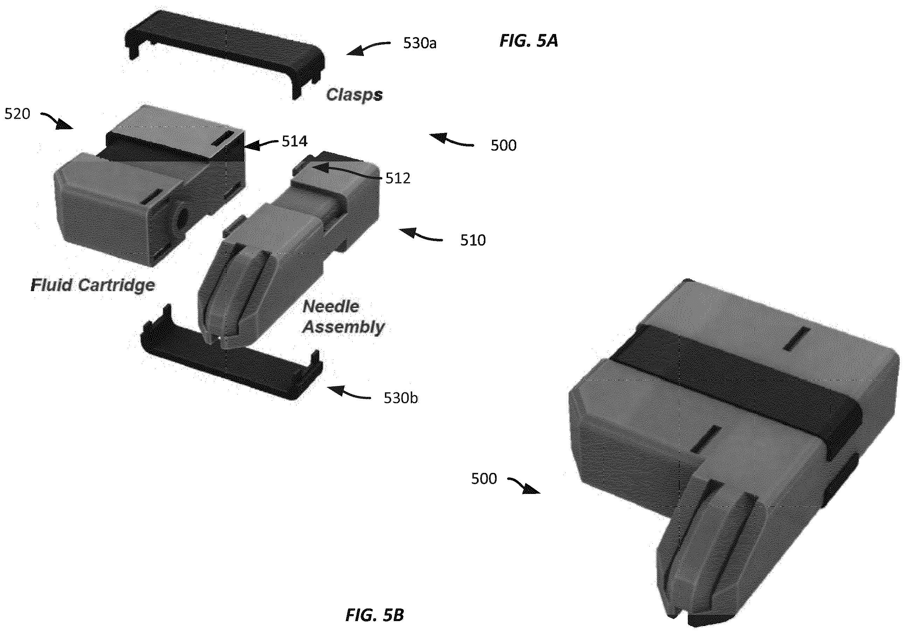

Referring now to Figures 5A-5B, Figure 5A shows another example of an injection portion 500 of an example wearable emergency drug injection device. In this example, the injection portion 500 includes a needle assembly 510 and a fluid cartridge 520 that are formed separately, but may be coupled together via one or more tabs 512 or one or more clasps 530a-b The example 500 shown in Figures 5A-5B includes both tabs 512 and clasps 530a-b. The tabs 512 engage with corresponding slots 514 to couple the fluid cartridge 520 with the needle assembly 510. The clasps 530a-b then engage with each of the fluid cartridge 520 and needle assembly 510 to create the injection portion of an example wearable emergency drug injection device. Figure 5B shows the injection portion 500 after it has been fully assembled.

It should be appreciated that in some examples, the needle assembly 510 may not be coupled to a separate fluid cartridge. Instead, the example needle assembly 510 may be a stand-alone injection portion with only a single chamber in which an injectable material may be disposed. In one such example, activation of the propellant behind a piston in the needle assembly 510 may both force a needle into a wearer of the device, but may also force the injectable material through the needle and into the wearer, thereby obviating the need for a separate fluid cartridge 520.

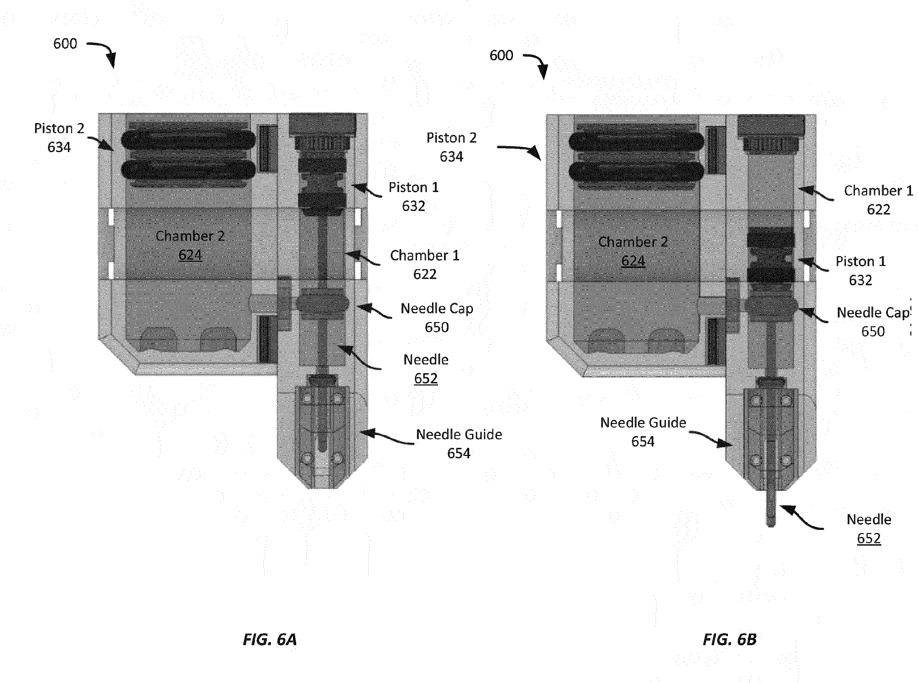

Referring now to Figures 6A-6D, these figures show an example injection portion 600 at different times during activation. Figure 6A shows the injection portion before it has been activated. The two pistons 632, 634 are at rest at one end of the respective chamber 622, 624. The needle cap 650 seals a fluid port between the two chambers 622, 624, and the needle 652 is entirely contained within the injection portion 600.

Referring now to Figure 6B, propellant behind piston 1 (632) has been activated, which as driven piston 1 (632) across chamber 1 (622) into contact with the needle cap 650. This movement has forced the needle 652 through the needle guide 654, which bends the needle 652. The propellant behind piston 2 (634) has not yet been activated at this point.

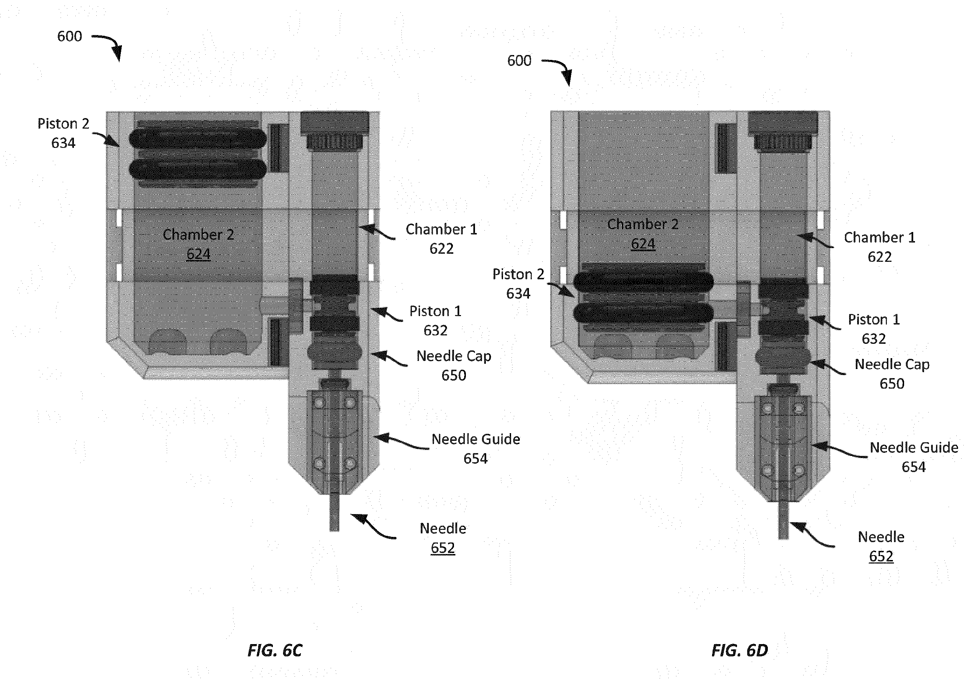

Referring now to Figure 6C, piston 1 (632) has continued to traverse chamber 1 and has driven needle cap 650 against the needle guide 654, thereby unsealing the fluid port between the two chambers 622, 624. An opening in piston 1 (632) aligns with the fluid port, providing a fluid path from chamber 2 (624) through the fluid port, the void in piston 1 (632) and into the needle 652. At this time, however, the propellant behind piston 2 (634) has not yet been activated, thus the contents of chamber 2 (624) have not yet been forced through the fluid port by piston 2 (634).

Referring now to Figure 6D, the propellant behind piston 2 (634) has been activated, which drove piston 2 (634) to the opposite end of chamber 2 (624), thereby forcing the contents of chamber 2 (624) through the fluid port and ultimately into and through the needle 652. Once piston 2 (634) has reached the far end of chamber 2 (624), the injection portion 600 has completed its operation, absent the use of a needle retraction mechanism, such as a spring or other biasing material or element.

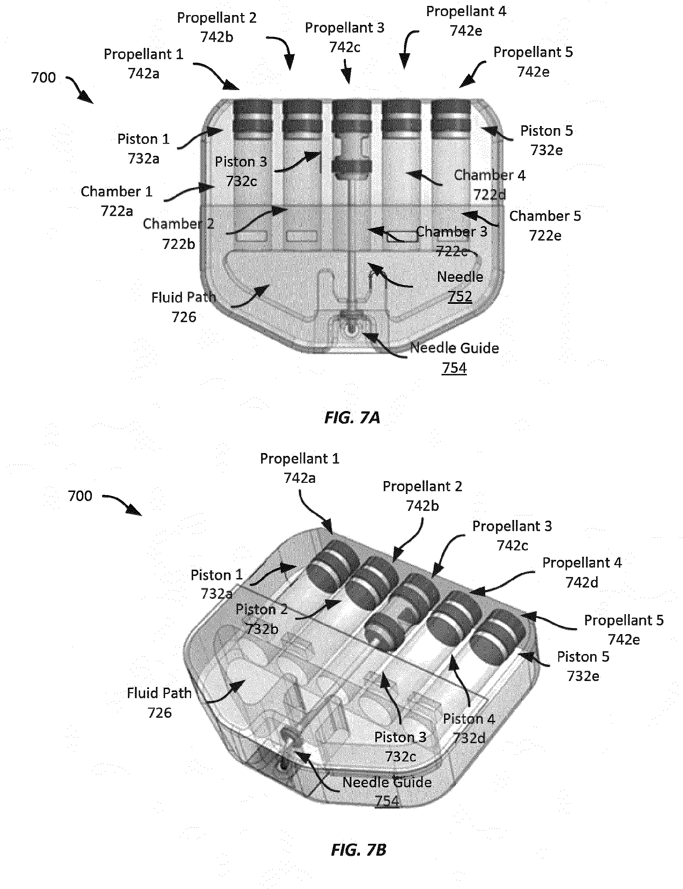

Referring now to Figures 7A-7B, Figure 7A shows an example wearable emergency drug injection device 700. In this example, the device 700 includes five separate chambers 722a-e, each having its own respective piston 732a-e and a corresponding propellant 742a-e. Each chamber 722a-e is coupled to a fluid path 726 which enables material from the respective chambers to be expelled from the chamber, through an opening in the third piston 732c and the hollow needle 752, thereby injecting the substance into the wearer. Figure 7B shows an alternate perspective of the device 700. Example devices having more than two chambers may be employed to dispense larger amounts of material, or may allow doses to be dispensed overtime. For example, the third propellant 742c may be activated to drive the third piston 732c across its chamber 722c, thereby driving the needle 752 through the needle guide 754 and into the wearer. One or more of the other propellant charges 742a,b,d,e may be activated to expel a substance through the fluid path and the needle into the wearer. Such charges may be activated in sequence or only when a respective dose of material is needed. And while this example includes five chambers, any suitable number of chambers may be employed according to various examples.

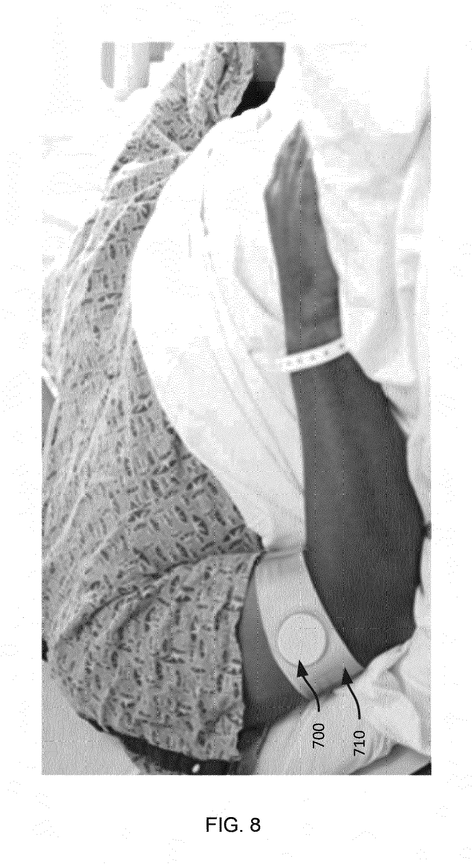

Referring now to Figure 8, Figure 8 shows an example application of a wearable emergency drug injection device 800 (or device 800) according to this disclosure. As discussed above, the device 800 may be worn by a person to enable on-demand injection of an injectable material in to the wearer. In this example, the device 800 is attached to an armband 810, which the wearer has wrapped around their upper arm. Thus, if the device 800 is activated, such as by a wireless signal from a remote device or based on wearer interaction with a manipulandum on the device 800 or armband 810, the device 800 may inject a needle into the wearer's arm and inject the injectable material through the needle, thereby delivering a dose of the injectable material.

While the example shown in Figure 8 is of an armband embodiment, other example wearable configurations are within the scope of this disclosure. For example, the device 800 may be attached to a wristband, or adhered to the wearer by a tape or an adhesive applied to one side of the device 800. In some examples, the features of the device may be incorporated into another wearable device, such as an insulin pump, smartwatch, etc. Still further configurations to enable wearing of the device 800 against the wearer's skin are within the scope of this disclosure.

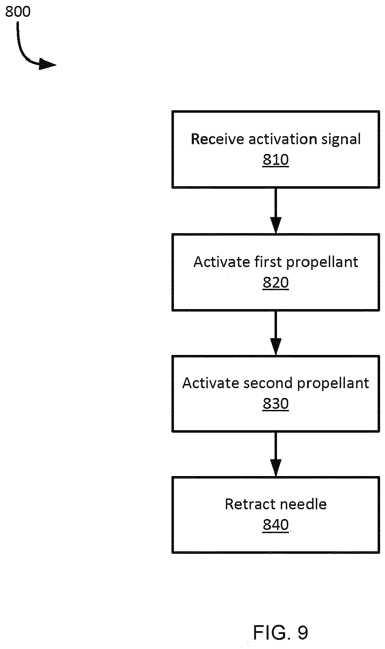

Referring now to Figure 9, Figure 9 shows an example method 900 for use of a wearable emergency drug injection device. The method 900 will be described with respect to the device 100 shown in Figure 1 and the system shown in Figure 2; however, any suitable device or system according to this disclosure may be employed, such as the example devices shown in Figures 3A-3G and 4A-4B.

At block 910, the device 100 receives an activation signal. In this example, the device 100 receives a wireless signal from the remote device 200 via a BLE wireless signal. To send the activation signal, the remote device 200 may first establish a communications connection with the device 100, such as by pairing with the device using the BLE protocol and then authenticating itself to the device 100, *e.g.,* by providing an encrypted communication comprising a digital signature or certificate. After establishing communications and authenticating itself to the device 100, the remote device 200 transmits an activation signal, which may comprise a command. And while in this example, the remote device 200 authenticates itself to the device 100, such a feature is not required. Instead, the remote device 200 may simply transmit an activation signal, such as by broadcasting an activation signal.

At block 920, in response to receiving the activation signal, the device 100 activates propellant 1 (142) to force piston 1 (132) towards the opposite end of chamber 1 (122). In this example, the firing circuit 112 closes a switch to discharge a capacitor onto an electrical contact coupled to propellant 1 (142). The electrical discharge from the capacitor ignites the propellant 142, which then burns or explodes. The piston 132 then traverses a portion of the chamber 122 and forces the needle through the needle guide 154. If a patient is wearing the device 100, the needle is also injected into the wearer's skin.

In some examples, a series of activation signals may be transmitted to propellant 1 (142). For example, propellant 1 (142) may include multiple propellant components, each of which may be individually activatable. By activating these propellant components in sequence, a suitable pressure curve may be generated over time.

At block 930, subsequent to activating the first propellant, the device 100 activates propellant 2 (144) to force piston 2 (134) towards the opposite end of the chamber 2 (124). In this example, the firing circuit 112 closes a switch to discharge another capacitor onto an electrical contact coupled to propellant 2 (144). The electrical discharge from the capacitor ignites the propellant 144, which then burns or explodes, generally as described above with respect to block 920. The piston 134 then traverses a portion of the chamber 122 and forces the needle through the needle guide 154. If a patient is wearing the device 100, injectable material in chambers 1 or 2 (122, 124) is dispensed into the wearer. As discussed above with respect to block 920, in examples where propellant 2 (144) includes multiple individually-activatable propellant components, multiple actuation signals maybe transmitted to activate the propellant components in sequence to create a suitable pressure curve for driving the piston.

At block 940, the device 100 retracts the needle 152. In this example, the springs 156a-b are compressed by the movement of the needle cap 150. After sufficient gasses have been exhausted from chamber 1 (122) and the chamber pressure drops below the force exerted by the springs 156a-b, the needle 152 is retracted. Still further needle retraction mechanisms may be employed in various examples. It should be appreciated that block 940 is optional. Thus, the needle 152 may not be automatically retracted by the device 100. Instead, the wearer may manually retract the needle 152, or may remove the device 100 to withdraw the needle 152 from their skin.

The foregoing description of some examples has been presented only for the purpose of illustration and description and is not intended to be exhaustive or to limit the disclosure to the precise forms disclosed. Numerous modifications and adaptations thereof will be apparent to those skilled in the art without departing from the spirit and scope of the disclosure.

Reference herein to an example or implementation means that a particular feature, structure, operation, or other characteristic described in connection with the example may be included in at least one implementation of the disclosure. The disclosure is not restricted to the particular examples or implementations described as such. The appearance of the phrases "in one example," "in an example," "in one implementation," or "in an implementation," or variations of the same in various places in the specification does not necessarily refer to the same example or implementation. Any particular feature, structure, operation, or other characteristic described in this specification in relation to one example or implementation may be combined with other features, structures, operations, or other characteristics described in respect of any other example or implementation.

Use herein of the word "or" is intended to cover inclusive and exclusive OR conditions. In other words, A or B or C includes any or all of the following alternative combinations as appropriate for a particular usage: A alone; B alone; C alone; A and B only; A and C only; B and C only; and A and B and C.

Aspects of the present disclosure may be expressed in the following clauses:
1. A device for injecting a substance into a patient, comprising: a housing defining at least a first chamber and a second chamber; an injector disposed within the housing and comprising: a hollow needle corresponding to a first piston, the first piston disposed and translatable within the first chamber towards a first end of the first chamber, the first piston defining a void intersecting a hollow portion of the hollow needle; a first propellant disposed within the first chamber and positioned to force the first piston towards the first end of the first chamber in response to activation of the first propellant; a dispenser disposed within the housing and comprising: a second piston disposed and translatable within the second chamber towards a first end of the second chamber; a second propellant disposed within the second chamber and positioned to force the second piston towards the first end of the second chamber in response to activation of the second propellant; and wherein translation of the first piston to the first end of the first chamber exposes the void to the first end of the second chamber.
2. The device of clause 1, further comprising a first propellant activator and a second propellant activator, the first propellant activator positioned proximate the first propellant and configured to activate the first propellant, the second propellant activator positioned proximate the second propellant and configured to activate the second propellant.
3. The device of clause 1 or 2, wherein the first and second propellants comprise nitrocellulose and the first and second propellant activators comprise resistors.
4. The device of any one of clauses 1 to 3, wherein the first propellant comprises nitrocellulose in a cotton format and the second propellant comprises nitrocellulose in a paper format.
5. The device of any one of clauses 1 to 4, wherein the housing is configured to be worn by a patient.
6. The device of clause 5, further comprising a strap, and wherein the housing is configured to strapped to a patient's arm or torso.
7. The device of any one of clauses 1 to 6, further comprising epinephrine disposed within the second chamber.
8. The device of clause 7, wherein the epinephrine comprises approximately 0.3 millilitres of epinephrine.
9. The device of clause 7, wherein the epinephrine comprises approximately 1 millilitre ("ml") of epinephrine, and further comprising: a piston stop disposed within the second chamber, the piston stop configured to halt movement of the second piston towards the first end of the second chamber after approximately 0.3 ml of epinephrine have been dispensed through the hollow needle.
10. The device of any one of clauses 1 to 6, further comprising a glucagon diluting solution disposed within the second chamber, and a glucagon powder disposed within the void.
11. The device of clause 10, wherein the glucagon diluting solution comprises approximately 1 ml of glucagon diluting solution and wherein the glucagon powder comprises approximately 1 milligram of glucagon powder.
12. The device of any one of clauses 1 to 11, wherein the hollow needle comprises a flexible 22-gauge (0.64 mm diameter) hollow needle and is approximately 2.5 cm (1 inch) long.
13. The device of clause 12, wherein the hollow needle is constructed from Nitinol.
14. The device of any one of clauses 1 to 13, further comprising a needle retraction mechanism.
15. The device of clause 14, wherein the needle retraction mechanism comprises a spring.
16. The device of any one of clauses 1 to 15, wherein the housing is a first housing, and further comprising: a second housing releasably couplable to the first housing; a battery disposed within the second housing; a first switch electrically coupling the battery and the first propellant, the first switch arranged to provide an electrical current to the first propellant when the first switch is closed; and a second switch electrically coupling the battery and the second propellant, the second switch arranged to provide an electrical current to the second propellant when the second switch is closed.
17. The device of clause 16, wherein: the first switch is coupled to the battery via a first capacitor, the first capacitor configured to store a first electrical charge when the first switch is open, and to discharge the first electrical charge to the first propellant when the first switch is closed; and the second switch is coupled to the battery via a second capacitor, the second capacitor configured to store a second electrical charge when the second switch is open, and to discharge the second electrical charge to the second propellant when the second switch is closed.
18. The device of clause 16, further comprising a manipulandum coupled to the first and second switches, the manipulandum configured to initiate a firing sequence in response to an input.
19. The device of clause 18, wherein the firing sequence comprises: closing the first switch; and subsequent to closing the first switch, closing the second switch.
20. The device of clause 16, further comprising a wireless receiver and an antenna, the wireless receiver in communication with the first switch and the second switch and configured to initiate a firing sequence in response to receipt of a firing signal.
21. The device of clause 20, wherein the firing sequence comprises: closing the first switch; and subsequent to closing the first switch, closing the second switch.
22. A method comprising: receiving an activation signal; in response to receiving the activation signal, activating a first propellant of an injection device to force a first piston towards a first end of a first chamber, the injection device comprising a hollow needle corresponding to the first piston, the first piston disposed and translatable within the first chamber towards the first end of the first chamber, the first piston defining a void intersecting a hollow portion of the hollow needle, and a second piston disposed and translatable within a second chamber towards a first end of the second chamber; subsequent to activating the first propellant, activating a second propellant of the injection device to force the second piston towards the first end of the second chamber, an injectable substance disposed within the second chamber; and wherein translation of the first piston to the first end of the first chamber exposes the void to the first end of the second chamber.
23. The method of clause 22, further comprising receiving a user input from a manipulandum coupled to the injection device, wherein the activation signal is received in response to receiving the user input.
24. The method of clause 22 or 23, further comprising receiving the activation signal from a remote device.
25. The method of clause 24, wherein the activation signal is received wirelessly from the remote device.
26. The method of clause 24, wherein the remote device comprises a continuous glucose monitor or an insulin pump.
27. The method of any one of clauses 22 to 26, further comprising retracting the hollow needle.
28. The method of any one of clauses 22 to 27, wherein the injectable substance comprises epinephrine.
29. The method of clause 28, wherein the epinephrine comprises approximately 0.3 ml of epinephrine.
30. The method of clause 28, wherein the epinephrine comprises approximately 1 ml of epinephrine, and further comprising: a piston stop disposed within the second chamber, the piston stop configured to halt movement of the second piston towards the first end of the second chamber after approximately 0.3 ml of epinephrine have been dispensed through the hollow needle.
31. The method of any one of clauses 22 to 27, wherein the injectable substance comprises a glucagon diluting solution disposed within the second chamber, and a second injectable substance comprises a glucagon powder disposed within the void.
32. The method of clause 31, wherein the glucagon diluting solution comprises approximately 1 ml of glucagon diluting solution and wherein the glucagon powder comprises approximately 1 milligram of glucagon powder.
33. A device for injecting a substance into a patient, comprising: a housing defining at least one chamber; and an injector disposed within the housing and comprising: a hollow needle corresponding to a piston, the piston disposed and translatable within the chamber towards a first end of the chamber, the piston defining a void intersecting a hollow portion of the hollow needle; and a propellant disposed within the chamber and positioned to force the piston towards the first end of the chamber in response to activation of the propellant.
34. The device of clause 33, further comprising a propellant activator, the propellant activator positioned proximate the propellant and configured to activate the propellant.
35. The device of clause 34, wherein the housing is configured to be worn by a patient.

## Claims

1. A wearable device for injecting a substance into a patient, comprising:
a housing defining at least one chamber; and
an injector disposed within the housing and comprising:
a hollow needle corresponding to a piston, the piston disposed and translatable within the chamber towards a first end of the chamber, the piston defining a void intersecting a hollow portion of the hollow needle;
a needle guide configured to bend the hollow needle in response to movement of the hollow needle through the needle guide; and
a propellant disposed within the chamber and positioned to force the piston towards the first end of the chamber in response to activation of the propellant.

2. The wearable device of claim 1, further comprising a propellant activator, the propellant activator positioned proximate the propellant and configured to activate the propellant.

3. The wearable device of claim 2, wherein the propellant activator comprises a resistor.

4. The wearable device of any of claims 1 to 3, wherein the propellant comprises nitrocellulose.

5. The wearable device of any of claims 1 to 4, further comprising an injectable substance disposed within the chamber.

6. The wearable device of any of claims 1 to 5, wherein the injectable substance comprises epinephrine.

7. The wearable device of any of claims 1 to 6, further comprising a needle retraction mechanism.

8. The wearable device of any of claims 1 to 7, wherein the housing is a first housing, and further comprising:
a second housing releasably couplable to the first housing;
a battery disposed within the second housing;
a switch electrically coupling the battery and the propellant, the switch arranged to provide an electrical current to the propellant when the switch is closed.

9. The wearable device of any of claims 1 to 7, wherein:
the housing defines at least two chambers, and
the chamber is a first chamber, the piston is a first piston, the propellant is a first propellant; and
wherein the wearable device further comprises:
a dispenser disposed within the housing and comprising:
a second piston disposed within a second chamber of the at least two chambers, the second piston translatable towards a first end of the second chamber;
a second propellant disposed within the second chamber and positioned to force the second piston towards the first end of the second chamber in response to activation of the second propellant; and
wherein translation of the first piston to the first end of the first chamber exposes the void to the first end of the second chamber.

10. The wearable device of claim 9, further comprising an injectable substance disposed within the second chamber.

11. The wearable device of claim 9, wherein the housing is a first housing, and further comprising:
a first propellant activator proximate the first propellant;
a second propellant activator proximate the second propellant;
a second housing releasably couplable to the first housing;
a battery disposed within the second housing;
a first switch electrically coupling the battery and the first propellant activator, the first switch arranged to provide an electrical current to the first propellant activator when the first switch is closed; and
a second switch electrically coupling the battery and the second propellant activator, the second switch arranged to provide an electrical current to the second propellant activator when the second switch is closed.

12. The wearable device of claim 11, wherein:
the first switch is coupled to the battery via a first capacitor, the first capacitor configured to store a first electrical charge when the first switch is open, and to discharge the first electrical charge to the first propellant when the first switch is closed; and
the second switch is coupled to the battery via a second capacitor, the second capacitor configured to store a second electrical charge when the second switch is open, and to discharge the second electrical charge to the second propellant when the second switch is closed.

13. The wearable device of claim 12, further comprising a manipulandum coupled to the first and second switches, the manipulandum configured to initiate a firing sequence in response to an input.

14. The wearable device of claim 12, further comprising a wireless receiver and an antenna, the wireless receiver in communication with the first switch and the second switch and configured to initiate a firing sequence in response to receipt of a firing signal.

15. The wearable device of claim 13 or 14, wherein the firing sequence comprises:
closing the first switch; and
subsequent to closing the first switch, closing the second switch.
